Europäisches Patentamt

European Patent Office

Office européen des brevets

(11) Veröffentlichungsnummer: **0 220 480**
**A1**

## (12) EUROPÄISCHE PATENTANMELDUNG

(21) Anmeldenummer: 86112929.4

(22) Anmeldetag: 18.09.86

(51) Int. Cl.⁴: **C 07 B 35/02,** C 07 C 5/03,
C 07 D 307/89

(30) Priorität: 01.10.85 DE 3534944

(43) Veröffentlichungstag der Anmeldung: 06.05.87
**Patentblatt 87/19**

(84) Benannte Vertragsstaaten: DE FR GB IT

(71) Anmelder: **BAYER AG, Konzernverwaltung RP
Patentabteilung, D-5090 Leverkusen 1 Bayerwerk (DE)**

(72) Erfinder: **Klösel, Gerhard, Eulenweg 13,
D-5653 Leichlingen (DE)**
Erfinder: **Kricsfalussy, Zoltan, Dr.,
Franz-Marc-Strasse 32, D-5090 Leverkusen 1 (DE)**
Erfinder: **Becker, Hans-Joachim, Dr.,
Elisabeth-Langgässer-Strasse 33,
D-5090 Leverkusen 1 (DE)**
Erfinder: **Kyri, Hans, Dr., Gerstenkamp 5,
D-5000 Köln 80 (DE)**

(54) **Katalytisches Hydrierverfahren.**

(57) C-C-Doppelbindungen werden mit Nickel enthaltenden Träger-Katalysatoren hydriert, die ein Trägermaterial enthalten, das erhalten wurde, indem man ein plastisches Gemisch enthaltend Kieselsäure, faserförmige Zellulose und ein alkalisches Kieselsol zunächst extrudiert oder einer Rollgranulierung unterwirft und anschließend trocknet und glüht.

EP 0 220 480 A1

BAYER AKTIENGESELLSCHAFT    5090 Leverkusen, Bayerwerk

Konzernverwaltung RP

Patentabteilung             Gai/by-c

### Katalytisches Hydrierverfahren

Die vorliegende Erfindung betrifft ein Verfahren zur katalytischen Hydrierung von C-C-Doppelbindungen in Gegenwart von Nickel-Trägerkatalysatoren, die ein spezielles Trägermaterial enthalten.

Katalytische Hydrierungen in Gegenwart von Nickel-Katalysatoren sind bekannt. Häufig werden solche Hydrierungen in Gegenwart von Raney-Nickel, d.h. fein verteiltem elementarem Nickel durchgeführt. Nachteilig hierbei ist, daß solche Hydrierungen normalerweise ein Lösungs- und/oder Verdünnungsmittel erfordern und der Katalysator wegen seiner geringen Korngröße schwierig vom Hydrierprodukt abtrennbar ist.

Tetrahydrophthalsäureanhydrid kann auch in Gegenwart von Trägerkatalysatoren hydriert werden. Die DE-OS 2 823 164 beschreibt eine solche Hydrierung in Gegenwart eines Katalysators, der Palladium, Ruthenium oder Nickel auf einem $Al_2O_3$-Träger enthält, wobei mindestens 20 % des $Al_2O_3$ in Li-Al-Spinell umgewandelt worden sind. Der Nachteil dieses Verfahrens besteht darin, daß der verwendete Katalysator

Le A 23 969

und der Träger relativ teuer sind, da vorzugsweise die Edelmetalle Palladium und Ruthenium eingesetzt werden.

Schließlich ist aus J.A.C.S. 60, 2145 (1938) bekannt, daß man Tetrahydrophthalsäureanhydrid gelöst in einem Lösungsmittel mit suspendiertem Raney-Nickel als Katalysator hydrieren kann. Der Nachteil dieses Verfahrens besteht darin, daß das Lösungsmittel und der Katalysator aus dem Hydriergut entfernt werden müssen.

Es wurde nun ein Verfahren zur katalytischen Hydrierung von C-C-Doppelbindungen in Gegenwart von Nickel enthaltenden Trägerkatalysatoren, die ein poröses Trägermaterial auf der Basis von Kieselsäure enthalten, gefunden, das dadurch gekennzeichnet ist, daß man Katalysatoren mit einem Trägermaterial einsetzt, das erhalten wurde, indem man ein plastisches Gemisch enthaltend gefällte und getrocknete, feinteilige Kieselsäure, faserförmige Zellulose und ein alkalisches Kieselsol zunächst extrudiert oder einer Rollgranulierung unterwirft und anschließend trocknet und glüht.

Das erfindungsgemäße Verfahren kann auf die katalytische Hydrierung verschiedenartiger C-C-Doppelbindungen angewendet werden. Die zu hydrierenden C-C-Doppelbindungen können beispielsweise sowohl in ketten- als auch in ringförmigen Molekülen enthalten sein. Vorzugsweise werden $C_1$- bis $C_{12}$-Alkene und $C_5$- bis $C_{12}$-Cycloalkene dem erfindungsgemäßen Verfahren unterworfen. Derartige Alkene und Cycloalkene können auch Substituenten tragen, vorzugsweise solche, die sich unter den Bedingungen einer katalytischen Hydrierung nicht in unerwünschter Weise verändern.

Le A 23 969

Besonders bevorzugt wird das erfindungsgemäße Verfahren auf die katalytische Hydrierung von Tetrahydrophthalsäureanhydrid und $C_1$- bis $C_8$-Alkyl-tetrahydrophthalsäureanhydriden zu den entsprechenden Hexahydrophthalsäureanhydriden angewendet, wobei in den Ausgangsverbindungen die Doppelbindung und die Alkylgruppe(n) jeweils an beliebigen Stellen lokalisiert sein können. Speziell wird das erfindungsgemäße Verfahren auf die Hydrierung von 4-Tetrahydrophthalsäureanhydrid zu Hexahydrophthalsäureanhydrid und auf die Hydrierung von 5-$C_1$- bis $C_4$-Alkyl-4-tetrahydrophthalsäureanhydriden zu 5-$C_1$- bis $C_4$-Alkyl-hexahydrophthalsäureanhydriden angewendet, insbesondere auf die Hydrierung von 4-Tetrahydrophthalsäureanhydrid und 5-Methyl-4-tetrahydrophthalsäureanhydrid.

Die in das erfindungsgemäße Verfahren einzusetzenden Verbindungen sind bekannt und auf bekannte Weise herstellbar. Beispielsweise kann man 4-Tetrahydrophthalsäureanhydrid und 5-Methyl-4-tetrahydrophthalsäureanhydrid aus Butadien bzw. Isopren und Maleinsäureanhydrid in einer Diels-Alder-Reaktion erhalten.

Die erfindungsgemäß einzusetzenden Katalysatoren zeichnen sich durch ein spezielles Trägermaterial aus. Dieses ist erhältlich, indem man ein plastisches Gemisch, das gefällte und getrocknete, feinteilige Kieselsäure, faserförmige Zellulose und ein alkalisches Kieselsol enthält, zunächst extrudiert oder einer Rollgranulierung unterwirft und anschließend trocknet und glüht. Die gefällte und getrocknete, feinteilige Kieselsäure weist vorzugsweise eine BET-Oberfläche von 20 bis 300 $m^2$/g, einen $SiO_2$-Gehalt von

Le A 23 969

über 85 Gew.-% und eine mittlere Korngröße von weniger als 0,001 mm auf. Eine geeignete Kieselsäure ist z.B. unter der Bezeichnung Durosil ® im Handel erhältlich.

Die faserförmige Zellulose wird bei der Herstellung des Trägermaterials vorzugsweise in dem alkalischen Kieselsol dispergiert. Die Menge der Zellulosefasern kann beispielsweise 0,1 bis 2,5 Gew.-%, bezogen auf die gesamte Rohstoffmischung zur Herstellung des Trägermaterials, oder 0,2 bis 5 Gew.-%, bezogen auf das eingesetzte alkalische Kieselsol, betragen. Die mittlere Länge der Zellulosefasern entspricht vorzugsweise mindestens dem halben Durchmesser des fertigen Trägermaterials. Die Zellulosefasern können beispielsweise in Form von Holzschliff, faserigen Zellulosepräparaten, faserigen regenerierten Zellulosen, Baumwolle oder weitgehend in Einzelfasern zerfallenden Zellulosefasern enthaltendem Papierbrei eingesetzt werden.

Das alkalische Kieselsol ist vorzugsweise durch einen Zusatz von beispielsweise Alkalihydroxid oder Alkalisilikat stabilisiert und weist vorzugsweise einen pH-Wert von mindestens 9 auf. Besonders bevorzugt sind alkalische Kieselsole, die mit Kaliumhydroxid oder Kaliwasserglaslösung stabilisiert sind, insbesondere mit solchen Mengen, daß das Gewichtsverhältnis von $K_2O:SiO_2$ im Bereich von 1:20 bis 1:200 liegt. Der Gehalt des Kieselsols an $SiO_2$ beträgt vorzugsweise 15 bis 35 Gew.-%. Das alkalische Kieselsol kann, bezogen auf die gefällte und getrocknete feinteilige Kieselsäure, beispielsweise in Mengen von 100 bis 200 Gew.-% eingesetzt werden.

Das plastische Gemisch, aus dem das erfindungsgemäß zu verwendende Trägermaterial hergestellt wird, kann gegebenenfalls weitere Komponenten enthalten, beispielsweise tonartige Substanzen, Füllstoffe und/oder rückgeführtes, gemahlenes Trägermaterial.

Bei den tonartigen Substanzen kann es sich z.B. um Ton, Kaolin, Bentonit, Attapulgit und/oder Hektorit handeln. Solche Substanzen können zusammen mit der gefällten und getrockneten, feinteiligen Kieselsäure eingesetzt werden. Die Menge tonartiger Substanz kann beispielsweise bis zu 50 Gew.-%, bezogen auf die Summe von gefällter und getrockneter, feinteiliger Kieselsäure und tonartiger Substanz, betragen.

Bei den Füllstoffen kann es sich z.B. um Kieselgur, Talkum oder feinstgemahlenen Glimmer handeln. Füllstoffe können ebenfalls zusammen mit der gefällten und getrockneten, feinteiligen Kieselsäure und gegebenenfalls zusätzlich zu tonartigen Substanzen eingesetzt werden. Die Menge Füllstoffe kann beispielsweise bis zu 25 Gew.-%, bezogen auf die Summe von gefällter und getrockneter, feinteiliger Kieselsäure, tonartiger Substanz und Füllstoffe, betragen.

Trägermaterialien für Katalysatoren müssen im allgemeinen eine bestimmte Korngröße oder Korngrößenverteilung aufweisen. Bei der Herstellung von Trägermaterialien fallen jedoch häufig auch größere und kleinere Teilchen an (sog. Über- und Unterkorn), die dann z.B. durch Aussieben abgetrennt und im allgemeinen verworfen werden. Bei der

**Le A 23 969**

Herstellung von erfindungsgemäß einzusetzendem Trägermaterial kann das abgetrennte Über- und Unterkorn gegebenenfalls gemahlen und in beliebigen Mengen der gefällten und getrockneten, feinteiligen Kieselsäure zugegeben werden. Beispielsweise kann diese Menge bis zu 50 Gew.-%, bezogen auf die Summe von Kieselsäure und tonartiger Substanz, betragen.

Das zuvor beschriebene plastische Gemisch kann auf an sich bekannte Weise extrudiert oder einer Rollgranulation unterworfen und anschließend getrocknet und geglüht werden, um das erfindungsgemäß einzusetzende Trägermaterial zu erhalten (siehe z.B. DE-OS 34 18 748). Gegebenenfalls sind dann noch die Teilchen mit der gewünschten Korngröße oder dem gewünschten Korngrößenbereich abzutrennen. Für die Trocknung werden vorzugsweise Temperaturen unter 100° C angewendet, für das Glühen vorzugsweise solche im Bereich von 500 bis 1000° C. Wenn man mit fest angeordnetem Katalysator arbeiten möchte ist ein geeigneter Korngrößenbereich beispielsweise ein solcher von 1 bis 3 mm Durchmesser. Für das Arbeiten mit suspendiertem Katalysator ist beispielsweise ein Korngrößenbereich von 0,1 bis 1,5 mm Durchmesser geeignet.

Vor der Durchführung des erfindungsgemäßen Verfahrens ist auf das zuvor beschriebene Trägermaterial noch Nickel aufzubringen. Dies kann auf an sich bekannte Weise geschehen, beispielsweise indem man das Trägermaterial zunächst mit einer Säure, wie Essigsäure oder Salzsäure, und danach mit Wasser wäscht und trocknet, anschließend eine Lösung einer Nickelverbindung, vorzugsweise eine wäßrige Lösung eines Nickelsalzes wie Nickelformiat oder Nickelnitrat in der gewünschten Menge auf das Trägermaterial auftränkt,

<u>Le A 23 969</u>

dann trocknet und schließlich die aufgetränkte Nickelverbindung reduziert. Die letzterwähnte Trocknung und die Reduktion kann beispielsweise bei Temperaturen von 200 bis 350° C durchgeführt werden, ein geeignetes Reduktionsmittel ist beispielsweise Wasserstoff. Der fertige Katalysator kann beispielsweise 0,5 bis 8 Gew.-% Nickel enthalten. Vorzugsweise enthält er 1 bis 5 Gew.-% Nickel.

Die erfindungsgemäße Hydrierung wird im allgemeinen ohne Zusätze von Lösungsmitteln und ohne die Rückführung bereits hydrierten Materials durchgeführt. Das Einsatzmaterial wird im allgemeinen in flüssiger Form ohne irgendwelche Zusätze der erfindungsgemäßen Hydrierung unterworfen.

Die Art der Hydrierung kann verschieden sein. Beispielsweise kann man den Katalysator fest anordnen und eine Riesel- oder Flutphase-Hydrierung durchführen. Man kann die Hydrierung auch mit suspendiertem Katalysator durchführen. Erfolgt die Hydrierung in der Rieselphase, so rieselt das zu hydrierende Substrat im Reaktor von oben nach unten über den Katalysator, der sich in einer Wasserstoffatmosphäre befindet. Der Wasserstoff kann von oben oder von unten in den Reaktor eingeführt werden. Führt man die Hydrierung in der Flutphase durch, so durchströmen Einsatzprodukt und Wasserstoff das Katalysatorbett von unten nach oben. Die Hydrierdauer in der Rieselphase ist von der Länge des Reaktors abhängig. Bei der Flutphase ist die Hydrierdauer abhängig von der Einsatzmenge und der Reaktorlänge.

Le A 23 969

Zweckmäßigerweise wird die Hydrierung bei Temperaturen durchgeführt, die oberhalb des Schmelzpunktes des jeweiligen Einsatzmaterials liegen und 250°C nicht übersteigen. Vorzugsweise wird bei Temperaturen im Bereich 135 bis 220°C gearbeitet, besonders bevorzugt im Bereich 150 bis 200°C. Der Druck während der Hydrierung beträgt im allgemeinen mindestens 10 bar. Bei höheren Drucken verläuft die Hydrierung normalerweise schneller. Bei Drucken über 200 bar werden im allgemeinen keine besonderen Vorteile mehr erhalten. Bevorzugt arbeitet man deshalb bei Drucken im Bereich von 50 bis 200 bar.

Die erfindungsgemäße Hydrierung kann ansatzweise oder kontinuierlich betrieben werden. Vorzugsweise arbeitet man kontinuierlich.

Als Hydriergas kann Wasserstoff oder ein Wasserstoff enthaltendes Gas verwendet werden, das vorzugsweise mindestens 20, besonders bevorzugt mindestens 50 Vol.-% Wasserstoff enthält. Eventuell vorhandene Katalysatorgifte werden zweckmäßigerweise vor der Hydrierung aus dem Hydriergas entfernt. Das den Hydrierreaktor nach Durchführung des erfindungsgemäßen Verfahrens verlassende Produkt ist im allgemeinen so rein, daß es keinen besonderen Aufarbeitungsmaßnahmen, wie Destillation oder Kristallisation, unterworfen werden muß, um ein weiterverwendbares Produkt zu erhalten. Wenn die Hydrierung mit suspendiertem Katalysator durchgeführt wurde ist dieser natürlich abzutrennen, z.B. durch Filtration oder Sedimentation.

Das erfindungsgemäße Verfahren hat eine Reihe von unerwarteten Vorteilen . Es kann mit einfach herzustellenden,

Le A 23 969

preisgünstigen Katalysatoren durchgeführt werden, obwohl bisher gute Ergebnisse nur mit Edelmetall- oder Raney-Katalysatoren erzielt wurden; es erfordert keine Lösungsmittel, d.h. die Raum-Zeit-Ausbeuten sind günstig und es entsteht kein Aufwand für die Abtrennung des Hydrierproduktes von Lösungsmitteln; soweit erforderlich, z.B. bei Suspensionshydrierung, ist der Katalysator und das Hydrierprodukt auf einfache Weise voneinander zu trennen; die Umsätze sind hoch, häufig nahezu quantitativ und die Selektivitäten gut, häufig nahe 100 %. Insgesamt betrachtet ist die erfindungsgemäße Hydrierung also besonders einfach und wirtschaftlich.

Die erfindungsgemäß erhältlichen Hydrierprodukte sind bekannte Verbindungen mit bekannten Verwendungsmöglichkeiten. Beispielsweise können Hexahydrophthalsäureanhydrid und 5-Methyl-hexahydrophthalsäureanhydrid, die erfindungsgemäß aus Tetrahydrophthalsäureanhydrid bzw. 5-Methyltetrahydrophthalsäureanhydrid zugänglich sind, als Härter für Polymere eingesetzt werden.

Die folgenden Beispiele erläutern die vorliegende Erfindung ohne sie in irgendeiner Weise zu beschränken.

Le A 23 969

**Beispiele**

**Beispiel 1**

In einem Eirichmischer von 150 Liter Rauminhalt wurden
9 kg Durosil[®] vorgelegt. Es wurden 14,6 kg Bindemittel,
bestehend aus 13,3 kg 30%igem Kieselsol 300F/30 und
1,1 kg einer 25%igen Kaliwasserglaslösung, in welcher
0,17 kg Holzschliff dispergiert waren, zugegeben. Nach
20 Minuten intensiven Rührens war der Inhalt des Mischers
zu einem perlförmig runden Granulat von 0,1 bis 3,0 mm
Durchmesser agglomeriert. Es wurde 1 kg Durosil zugegeben
und der Rührvorgang etwa 2 Minuten fortgesetzt.

Das fertige Granulat wurde entnommen und in einem Umlufttrockner bei 90°C bis zur Gewichtskonstanz getrocknet.

Durch Siebung wurden etwa 80 % der Gesamtmenge als Kornfraktion zwischen 0,8 und 2,0 mm Korndurchmesser gewonnen.
Das Unterkorn und das Überkorn wurden zurückgestellt und
nach der Vermahlung auf einer Stiftmühle bei einem neuen
Ansatz dem Durosil[®] zugesetzt. Die Fraktion 0,8 bis 2,0 mm
wurde 4 Stunden bei 600°C in einem Kammerofen geglüht.

Das so erhaltene Granulat wurde mit Essigsäure gewaschen
und gespült. Danach wurde eine wäßrige Lösung von Nickelformiat in einem Rotationsverdampfer aufgetränkt und das
Gemisch bei 300°C getrocknet. Danach wurde bei der gleichen Temperatur mit Wasserstoff reduziert. Der fertige
Katalysator wies eine BET-Oberfläche von 70 m$^2$/g auf. Das

Le A 23 969

Gesamtporenvolumen betrug 421 mm²/g, der mittlere Porendurchmesser 240 A, die aufgebrachte Nickelmenge 1,4 Gew.-%.

Beispiel 2

In einen rohrförmigen Reaktor mit einer Länge von 0,5 m und einer lichten Weite von 40 mm wurden 580 ml des gemäß Beispiel 1 hergestellten Katalysators eingefüllt. Das Rohr war mit sechs separat regelbaren elektrischen Widerstandsheizungen versehen, die mit Luft gekühlt werden konnten. Der Katalysator war in dem Reaktionsrohr fest angeordnet. Über den Katalysator wurde von oben geschmolzenes 4-Tetrahydrophthalsäureanhydrid und Wasserstoff geleitet. Am unteren Ende des Reaktors befand sich ein Abscheider, in dem das flüssige Reaktionsprodukt vom Restgas abgetrennt wurde. Bei einer Temperatur von 160°C und einem Druck von 75 bar wurde das 4-Tetrahydrophthalsäureanhydrid kontinuierlich hydriert. Die Ausbeute an Hexahydrophthal-säureanhydrid betrug 98,4 %, die Raum-Zeit-Ausbeute 344 g pro Liter Katalysator und Stunde, die Selektivität 99 %.

Beispiel 3

Es wurde verfahren wie in Beispiel 2, jedoch wurde die Hydrierung bei 175°C und 90 bar durchgeführt. Die Ausbeute an Hexahydrophthalsäureanhydrid betrug 98,2 %, die Raum-Zeit-Ausbeute 415 g pro Liter Katalysator und Stunde, die Selektivität 98 %.

Le A 23 969

0220480

## Beispiel 4

Es wurde verfahren wie in Beispiel 2, jedoch wurde 5-Methyl-4-tetrahydrophthalsäureanhydrid bei 170°C und 75 bar hydriert. Die Ausbeute an 5-Methyl-hexahydrophthalsäureanhydrid betrug 79,8 %, die Raum-Zeit-Ausbeute 94 g pro Liter Katalysator und Stunde, die Selektivität 93 %.

Le A 23 969

## Patentansprüche

1. Verfahren zur katalytischen Hydrierung von C-C-Doppelbindungen in Gegenwart von Nickel enthaltenden Trägerkatalysatoren, die ein poröses Trägermaterial auf der Basis von Kieselsäure enthalten, dadurch gekennzeichnet, daß man Katalysatoren mit einem Trägermaterial einsetzt, das erhalten wurde, indem man ein plastisches Gemisch enthaltend gefällte und getrocknete, feinteilige Kieselsäure, faserförmige Zellulose und ein alkalisches Kieselsol zunächst extrudiert oder einer Rollgranulierung unterwirft und anschließend trocknet und glüht.

2. Verfahren nach Anspruch 1, dadurch gekennzeichnet, daß man $C_1$- bis $C_{12}$-Alkene oder $C_5$- bis $C_{12}$-Cycloalkene hydriert.

3. Verfahren nach Ansprüchen 1 und 2, dadurch gekennzeichnet, daß man Tetrahydrophthalsäureanhydrid oder $C_1$- bis $C_8$-Alkyl-tetrahydrophthalsäureanhydride hydriert.

4. Verfahren nach Ansprüchen 1 bis 3, dadurch gekennzeichnet, daß man eine gefällte und getrocknete, feinteilige Kieselsäure einsetzt, die eine BET-Oberfläche von 20 bis 300 $m^2$/g, einen $SiO_2$-Gehalt von über 85 Gew.-% und eine mittlere Korngröße von weniger als 0,001 mm aufweist.

5. Verfahren nach Ansprüchen 1 bis 4, dadurch gekennzeichnet, daß faserförmige Zellulose in einer Menge

Le A 23 969

von 0,1 bis 2,5 Gew.-%, bezogen auf die gesamte Rohstoffmischung zur Herstellung des Trägermaterials, eingesetzt wird und die mittlere Länge der Zellulosefasern mindestens dem halben Durchmesser des fertigen Trägermaterials entspricht.

6. Verfahren nach Ansprüchen 1 bis 5, dadurch gekennzeichnet, daß das alkalische Kieselsol mit Alkalihydroxid oder Alkalisilikat stabilisiert wurde, einen pH-Wert von mindestens 9, und einen $SiO_2$-Gehalt von 15 bis 35 Gew.-% aufweist.

7. Verfahren nach Ansprüchen 1 bis 6, dadurch gekennzeichnet, daß das plastische Gemisch zusätzlich tonartige Substanzen, Füllstoffe und/oder rückgeführtes, gemahlenes Trägermaterial enthält.

8. Verfahren nach Ansprüchen 1 bis 7, dadurch gekennzeichnet, daß der fertige Katalysator 0,5 bis 8 Gew.-% Nickel enthält.

9. Verfahren nach Ansprüchen 1 bis 8, dadurch gekennzeichnet, daß man die Hydrierung bei 135 bis 220°C durchführt.

10. Verfahren nach Ansprüchen 1 bis 9, dadurch gekennzeichnet, daß man die Hydrierung bei Drucken im Bereich von 50 bis 200 bar durchführt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 005 738 (BAYER)<br>* Patentanspruch * | 1-10 | C 07 B 35/02<br>C 07 C 5/03<br>C 07 D 307/89 |
| A | DE-C- 961 622 (ALLIED CHEMICAL & DYE)<br>* Patentansprüche * | 1-10 | |
| A | DE-A-2 150 975 (ESSO RESEARCH & ENGINEERING)<br>* Patentansprüche * | 1-10 | |
| A | FR-A-1 023 462 (SOCIETE BELGE DE L'AZOTE ET DES PRODUITS CHIMIQUES DU MARLY)<br>* Zusammenfassung * | 1-10 | |
| A,P<br>D | DE-A-3 418 748 (BAYER)<br><br>* Patentansprüche *<br><br>----- | 1-10 | **RECHERCHIERTE SACHGEBIETE (Int. Cl.4)**<br><br>C 07 B 35/00<br>C 07 D 307/00 |

Der vorliegende Recherchenbericht wurde für alle Patentansprüche erstellt.

| Recherchenort<br>DEN HAAG | Abschlußdatum der Recherche<br>15-01-1987 | Prufer<br>WRIGHT M.W. |
|---|---|---|

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82